**Europäisches Patentamt**

**European Patent Office**

**Office européen des brevets**

(19)

(11) Veröffentlichungsnummer: **0 303 719**
**A1**

## EUROPÄISCHE PATENTANMELDUNG

veröffentlicht nach Art. 158 Abs. 3 EPÜ

(12)

(21) Anmeldenummer: **88902610.0**

(51) Int. Cl.⁴: **A 61 F 2/22**

(22) Anmeldetag: **15.02.88**

Daten der zugrundeliegenden internationalen Anmeldung:

(86) Internationale Anmeldenummer:
**PCT/SU 88/00036**

(87) Internationale Veröffentlichungsnummer:
**WO 88/06027 (25.08.88 88/19)**

(30) Priorität: **23.02.87 SU 4197835**

(43) Veröffentlichungstag der Anmeldung: **22.02.89**
**Patentblatt 89/8**

(84) Benannte Vertragsstaaten: **DE FR GB IT SE**

(71) Anmelder: **BLAGOVESCHENSKY GOSUDARSTVENNY MEDITSINSKY INSTITUT, UL: Gorkogo 97, Blagoveschensk, 675006 (SU)**

(72) Erfinder: **KULIK, Yaroslav Petrovich, ul. Zeiskaya, 140-36, Blagoveschensk, 675006 (SU)**

(74) Vertreter: **Ebbinghaus, Dieter et al, Patentanwälte v. Füner, Ebbinghaus, Finck Mariahilfplatz 2 & 3, D-8000 München 90 (DE)**

(54) **KÜNSTLICHER HERZBEUTEL.**

(57) Künstliches Perikard enthält eine Kapsel (1) aus einem biologisch neutralen Werkstoff in der Größe des jeweiligen Herzens, die mit einem Befestigungsmittel zu deren Festlegung am Herzen versehen ist. Die erwähnte Kapsel (1) weist die Gestalt eines 8förmigen Läppchens auf, dessen ein Oval (2) eine Oberfläche größer als das andere Oval (3) besitzt und mit einem Vorsprung in Form eines Zungenzäpfchens (4) am Läppchengipfel versehen ist. Das Befestigungsmittel ist als ein Führungskanal (5) an dem Läppchenumfang entlang, eine innerhalb des Führungskanals (5) eingesetzte Röhre (6) aus elastischem Werkstoff und ein innerhalb der Röhre (6) unter Ermöglichung dessen Hin- und Herbewegung untergebrachter Zugdraht (7) ausgeführt.

EP 0 303 719 A1

ACTORUM AG

KÜNSTLICHES PERIKARD

Technische Gebiet

Die vorliegende Erfindung bezieht sich auf das Gebiet Medizin, und zwar künstliche Organe, insbesondere betrifft sie künstliches Perikard.

Zugrundeliegender Stand der Technik

Bereits ist bekannt ein künstliches Perikard "Pery-Juard", das einen Lappen der Rechteckgestalt darstellt, der aus dem Gewebe des natürlichen Perikardes eines Stiers hergestellt wird, welches zwecks Vernichtung der Antigen.eigenschaften berarbeitet wird.

Das bekannte künstliche Perikard wird dann in Form eines Flickens zur Überdeckung eines eventuellen Fehlers des natürlichen Perikardes nach der Herzoperation verwendet. Jedoch bedeckt der Flicken hierbei nicht das ganze Herz und demzufolge ist die Entwicklung von Kommissuren durchaus möglich. Darüber hinaus wird das Heterogewebe des künstlichen "Stiersperikardes" vom Organismus abgestoßen und es entwickelt sich Kalzinose.

Bekannt ist auch ein künstliches Perikard, das Kapseln aus einem elastischen, biologisch neutralen Werkstoff in der Größe des Herzens einschließt, welche mit einem Befestigungsmittel zu deren Festlegung an dem Herzen ausgestattet werden (SU, A 1 009 457).

Die erwähnte Kapsel weist die Gestalt vom Herzen auf und ist an der Seite der Herzbasis geöffnet. Über die ganze Oberfläche der Kapsel sind Perforationslöcher ausge - spart. Das Befestigungsmittel des betreffenden bekannten Perikardes ist in Form von Tragstreifen seitens des offenen Endes der Kapsel ausgeführt. Nach der durchgeführten Herzoperation wird das Herz in die Höhle der Kapsel des künstlichen Perikardes hineingesetzt und mit den erwähnten, über den Sinus transversus verlegten Tragstreifen den Aortenbogen erfaßt, während die Streifenenden am Rande der Kapsel herangenäht werden. Nach den durchgeführten Manipulationen ist die Kapsel des künstlichen Perikardes am Herzen     sicher festgelegt, indem dadurch die ganze Oberfläche des Herzens unter Zustandebringen einer Interposition zwischen der Außenfläche des Herzens und der inneren Fläche des natürlichen Perikardes bedeckt wird; dabei kann zwischen diesen beiden keine Kommissurenbildung nachgewiesen werden. Die Perforationslöcher ermöglichen ungestörten flüssigkeitsbiologischen Stoffwechsel zwischen der Herzoberfläche und der Höhle des Perikardes, in der sich beispielsweise die seröse Flüssigkeit befindet, die das Gleiten der Herzwandung bei der Systole bewirkt.

Das künstliche Perikard der erwähnten Ausführung muß nach der Größe des Herzens ausgewählt werden, was es erfordert, eine große Anzahl der Größenarten einer optimalen Variante zu wählen. Die Fixierung des künstlichen Perikardes am Herzen vermittels der Herannähung der Tragstreifen an der Kapsel bringt jeweilige Unbequemlichkeiten bei dem arbeitenden Herzen mit sich. Darüber hinaus muß auch der Brustkorb zum Entfernen des künstlichen Perikardes nach der Beendigung der Nachoperationsperiode geöffnet werden, was eine Steigerung der Verletzbarkeit der Operation und Herausfließen der perikardialen Flüssigkeit bewirkt.

## Offenbarung der Erfindung

Der Erfindung liegt die Aufgabe zugrunde, eine Bauart des künstlichen Perikardes derart zu entwickeln, daß dadurch dessen Entfernung aus dem Brustkorb ohne Eröffnung des letzteren durchgeführt werden kann, wodurch die Verletzbarkeit der Operation herabgesetzt wird, jede Möglichkeit des Herausfließens der perikardialen Flüssigkeit aus der Höhle des natürlichen Perikardes ausgeschlossen wird und daß das künstliche Perikard selbst sich mit einem breiteren Mehrzweckanwendungsbereich und besserer Bequemlichkeit im Gebrauch auswirkt.

Das Wesen der Erfindung besteht darin, daß in dem eine mit dem Befestigungsmittel zu deren Festlegung am Herzen ausgerüstete Kapsel in der Größe des Herzens aus einem biologisch neutralen Werkstoff einschließenden, künstlichen Perikard erfindungsgemäß die Kapsel als ein 8-förmiges Läppchen ausgeführt ist, dessen ein Oval eine größere Fläche als das andere Oval aufweist und mit einem Vorsprung in Form von Zungenzäpfchen an dessen Gipfel versehen ist, während das Befestigungsmittel als ein Führungskanal am Läppchenumfang, eine in diesem Führungskanal hineingesetzte Röhre aus elastischem Werkstoff, deren Hin- und Herbewegung in diesem Kanal ermöglicht ist, und ein federnder, innerhalb der erwähnten elastischen Röhre untergebrachter Zugdraht ausgeführt ist, der ebenfalls seine Hin- - und Herbewegungsgänge in bezug auf die Röhre vollführen kann, wobei die Enden der Röhre und des Zugdrahts aus dem Führungskanal seitens des Vorsprunges herausgeführt sind.

Zwecks Steigerung der Bequemlichkeit bei der Herausnahme des künstlichen Perikardes ist die elastische Röhre zweckmäßigerweise aus zwei längengleichen Röhrenhälften auszuführen, während der Zugdraht auch in zwei Drahtteilen einzuteilen ist, von denen ein Drahtteil kürzer und der andere auf ein und dieselbe Größe länger als eine Röhrenhälfte ist.

Wünschenswert ist das Läppchen mit Haltern zur Fest- nalterung von Endoelektroden eines Kardiostimulators zu versehen.

Das erfindungsgemäß ausgeführte künstliche Perikard kann aus dem Brustkorb ohne dessen Eröffnung durch den Einschnitt im Oberabteil des natürlichen Prikardes bis 5mm und den Einschnitt im der Haut von 5 bis 6 mm Länge herausgenommen werden. Dabei kommt es zu keinem Verletzen des Herzens und der Umgebungsgewebe, darunter auch der Gewebe des natürlichen Perikardes, wodurch kein Herausfließen der Flüssigkeit aus der Höhle des natürlichen Perikardes zu- standekommt.

Das erfindungsgemäße künstliche Perikard bedarf erfindungsgemäß keiner sorgfältigen Auswahl dessen Größe, zeichnet sich durch seine breitere Mehrzweckanwendbarkeit und bessere Bequemlichkeit im Gebrauch aus, bedarf auch keine Anlegung der Festlegungsnähte zu dessen Festlegung am Herzen.

### Kurzbeschreibung der Zeichnungen

Nachstehend wird die Erfindung anhand der Beschreibung deren konkreter Ausführungsvariante und der angelegten Zeichnungen näher erläutert, in denen es zeigt

Fig. 1 erfindungsgemäßes künstliches Perikard in Draufsicht;

Fig. 2 dasselbe aus der Fig. 1 in Seitenansicht dessen Stellungslage der Festlegung am Herzen;

Fig. 3 dasselbe aus der Fig. 1 in Sterilpackung;

Fig. 4 Schnitt IV-IV auf der Fig. 3;

Fig. 5 das Herz und das erfindungsgemäße künstliche Perikard innerhalb der Höhe des natürlichen Perikardes in räumlicher Darstellung.

Bester Weg zur Ausführung der Erfindung

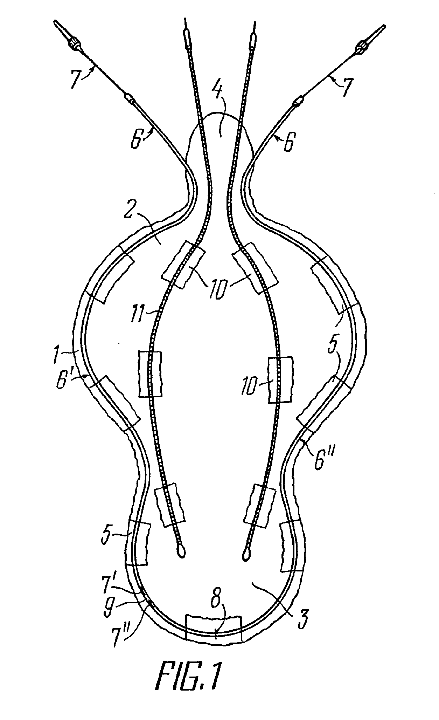

Das in der Fig. 1 wiedergegebene künstliche Perikard sicht eine Kapsel 1 aus einem biologisch neutralen, elastischen Werkstoff, beispielsweise aus Polyurethan und ein Befestigungsmittel zum Festlegen der erwähnten Kapsel 1 am Herzen vor. Die Kapsel 1 ist in Form eines 8-förmigen Läppchens ausgeführt, dessen ein Oval 2 eine größere Fläche als das andere Oval 3 besitzt. An dem Gipfel des größeren Ovals 2 ist ein Vorsprung in Form eines Zungenzäpfchens 4 vorgesehen. Das Befestigungsmittel schließt einen an dem Läppchensumfang verlegten Führungskanal 5, eine Röhre 6 aus einem elastischen Werkstoff, beispielsweise aus Polyurethan, die in den Führungskanal 5 eingesetzt wird und sich in diesem hin- und herbewegen kann, und einen längs der Achse des Kanals 5 innerhalb der Röhre 6 unter Ermöglichung dessen Hin- und Herbewegung in bezug auf die Röhre 6 untergebrachten, federnden Zugdraht 7 ein. Der Führungskanal 5 kann aus dessen einzelnen Strecken zusammengestellt werden, die in Längsrichtung des Läppchensumfanges verteilt sind, bzw. über die erwähnte Länge des Läppchensumfanges ungeteilt verlegt werden. Im allgemeinen wird der Führungskanal 5 aus demselben Werkstoff wie die Kapsel 1 hergestellt. In der Beschreibungsversion der Ausführung des künstlichen Perikardes besteht die Röhre 6 aus zwei längengleichen, mit ihren Enden im Punkt 8 am Gipfel des kleineren Ovals 3 aneinander anliegenden Röhrenhälften 6′ und 6′′; der federnde Zugdraht 7 ist ebenfalls aus zwei Drahtteilen 7′ und 7′′ unterschiedlicher Länge aber zusammengestellt, von denen der Drahtteil 7′ kürzer und der andere Drahtteil 7′′ auf ein und dieselbe Strecke als eine der Hälften 6'der Röhre 6 ist; infolgedessen stoßen die Enden der Drahtteile 7' und 7" des Zugdrahts 7 im Punkt 9 innerhalb der Röhrenhälfte 6' der Röhre 6 gegeneinander. Die anderen Enden der Röhre 6 und des Zugdrahts 7 ragen aus dem Führungskanal 5 seitens des Zungenzäpfchens 4 aus den Grenzen der Kapsel 1 hin heraus. Darüber hinaus werden an der Oberfläche der Kapsel 1 die Halter 10 mit in diesen festgelegten Endoelektroden 11 eines Kardiostimulators befestigt. Die erwähnten Halter 10 sind als kurze Kanäle ausgeführt, deren Querschnitt mit dem Durchmesser der jeweiligen Endoelektrode 11 vergleichbar ist, welche Kurzkanäle aus einem Polyurethanfilm hergestellt und auf der Oberfläche der Kapsel 1 befestigt sind.

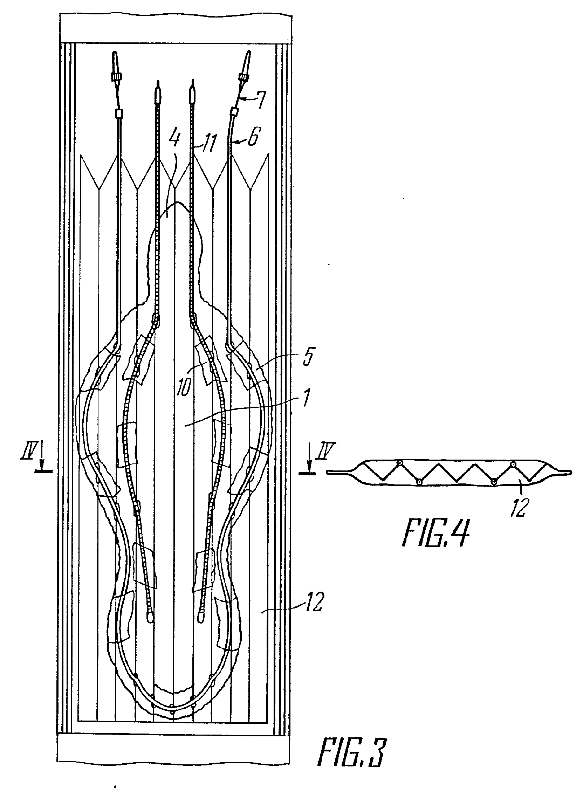

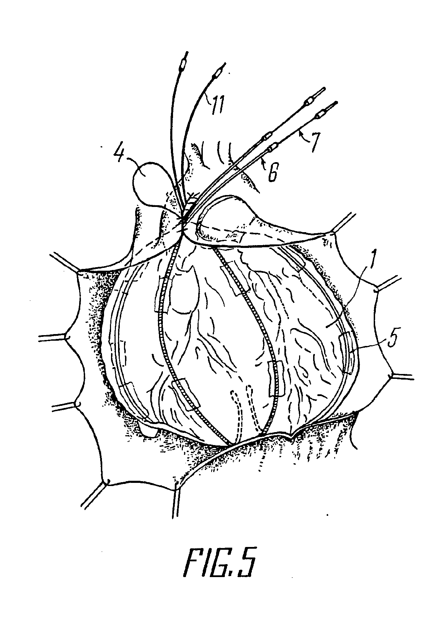

Bis auf Beginn der Operation wird das künstliche Perikard erfindungsgemäß in einer sterilen, luftdichten Packung 12 (Fig. 3, 4) aufbewahrt.

Das erfindungsgemäße künstliche Perikard wird wie folgt gebraucht.

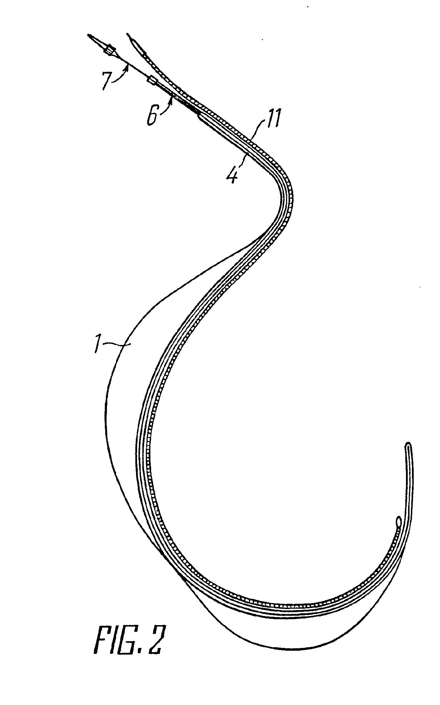

Nach der Beendigung einer korrigierenden Operation des Herzens wird das künstliche Perikard vor dem luftdichten Zusammennähen des natürlichen Perikardes aus der sterilen Packung 12 (Fig. 3) herausgenommen und unter die hintere Herzoberfläche mit seinem kleineren Oval 3 zu der Herzbasis hin (Fig. 1, 2) hinuntergelegt, während die vordere Oberfläche des Herzens und teilweise auch der Umgebungsgefäße mit dem größeren Oval 2 von vorne bedeckt wird. Dabei nimmt das künstliche Perikard seine Stellungslage ein, wie diese in Fig. 2 wiedergegeben ist. Hiernach wird das natürliche Perikard vollständig luftdicht zusammengenäht, indem aber in seinem Oberteil eine Wunde bis 5 mm lang vorhanden bleibt, durch welche das Zungenzäpfchen 4 (Fig. 5) des künstlichen Perikardes und die Enden der Röhre 6 und des Zugdrahts 7 herausgeführt und unter der Haut untergebracht werden. Die Haut wird aber hiernach zusammengenäht. In 10 bis 12 Tagen wird die Haut auf der Länge 5 bis 6 mm über das Zungenzäpfchen 4 des künstlichen Perikardes eingeschnitten. In diesen Einschnitt werden die Enden der Röhre 6, des federnden Zugdrahts 7 und das Zungenzäpfchen 4 herausgeführt.

00303719

Hiernach werden in einer bestimmten Reihenfolge die Teile 7´, 7" des Zugdrahts 7, dann die Hälften 6´ und 6" der Röhre 6 und am Zungenzäpfchen 4 auch die Kapsel 1 des künstlichen Perikardes herausgezogen.

Durch Verwendung des betreffenden künstlichen Perikardes wird es möglich, bei dessen Entfernung den Brustkorb nicht zu eröffnen, und dank dem erfindungsgemäßen Aufbau des künstlichen Perikardes ohne einer sorgfältigen Auswahl dessen Größe auszukommen. Bei der Herausnahme des künstlichen Perikardes werden das Herz und die Umgebungsgewebe keinesfalls verletzt. Es erübrigen sich die zusätzlichen Manipulationen beim Hineinsetzen des erfindungsgemäßen künstlichen Perikardes, die sonst zur Befestigung des Perikardes am Herzen durchgeführt werden, bei dessen Herausnahme wird Herausfließen der perikardialen Flüssigkeit aus der Höhle des natürlichen Perikardes vermieden.

Gewerbliche Verwendbarkeit

Die Erfindung ist zur Verwendung bei den Operationen am Herzen zum Vorbeugen den Kommissurvorgänge zwischen dem Herzen und natürlichen Perikard während der Nachoperationsperiode vorausbestimmt.

- 7 -                    00303719

PATENTANSPRÜCHE:

1. Künstliches Perikard, das eine Kapsel (1) in der Größe des Herzen aus einem biologisch neutralen Werkstoff einschließt, die mit einem Befestigungsmittel zu deren Festlegung am Herzen versehen ist, d a d u r c h  g e k e n n z e i c h n e t, daß die Kapsel (1) eine Form des 8-förmigen Läppchens aufweist, dessen ein Oval (2) mit der größeren Fläche als das andere Oval (3) und einem Vorsprung in Form des Zungenzäpfchens (4) an dessen Gipfel versehen ist, während das Befestigungsmittel als ein an dem Läppchensumfang entlang verlegter Führungskanal (5), eine innerhalb des Führungskanals (5) unter Ermöglichung deren Hin- und Herbewegung eingesetzte Röhre (6) aus elastischem Werkstoff und ein federnder, innerhalb der Röhre (6) unter Ermöglichung dessen Hin- und Herbewegung bezüglich der Röhre (6) untergebrachter Zugdraht (7) ausgeführt ist, wobei die Enden der Röhre (6) und des Zugdrahts (7) aus dem Führungskanal (5) seitens des Vorsprunges des Läppchens herausgeführt sind.

2. Künstliches Perikard nach Anspruch 1, d a d u r c h  g e k e n n z e i c h n e t, daß die Röhre (6) aus zwei längengleichen Röhrenhälften (6´, 6") zusammengestellt ist, während der Zugdraht (7) ebenfalls aus zwei Drahtteilen (7´, 7") besteht, von denen einer kürzer und der andere länger als eine der Hälften (6´) der Röhre (6) auf ein und dieselbe Strecke ausgeführt ist.

3. Künstliches Perikard nach Anspruch 1 bzw. 2, d a d u r c h  g e k e n n z e i c h n e t, daß das Läppchen mit den Haltern (10) zum Festhalten der Endoelektroden (11) eines Kardiostimulators versehen ist.

1/4

FIG.1

FIG.2

FIG.3

FIG.4

FIG.5

## I. CLASSIFICATION OF SUBJECT MATTER (if several classification symbols apply, indicate all) •

According to International Patent Classification (IPC) or to both National Classification and IPC

IPC⁴ _ A 61 F 2/22

## II. FIELDS SEARCHED

### Minimum Documentation Searched ⁷

| Classification System | Classification Symbols |
|---|---|
| IPC⁴ | A 61 F 2/22, A 61 H 31/00, A 61 B 17/34 |

Documentation Searched other than Minimum Documentation
to the Extent that such Documents are Included in the Fields Searched ⁸

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| A | SU,A1,1111752(Problemnaya nauchno-issledova-telskaya labotatoria "Vspomogatelnogo Krovoobraschenia" Blagoveschenskogo meditsinskogo instituta),07 September 1984 (07.09.84) -- | 1,2 |
| A | US,A,4497074(Agence National de Valorisation de la Recherche (ANVAR)),05 February 1985(05.02.85),see claims 1,2,15,drawing -- | 1 |
| A | US,A,4536893(Robert Parravicini),27 August 1985(27.08.85),see the claims,drawing -- | 1 |
| A | FR,A1,2519544(CASILE Jean Pierre)18 July 1983 (18.07.83),see claims 1,6,9,12,drawing ------- | 1-3 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 18 April 1988(18.04.88) | 25 May 1988(25.05.88) |
| International Searching Authority | Signature of Authorized Officer |
| ISA/SU | |

Form PCT/ISA/210 (second sheet) (January 1985)